# FASCICULE DE BREVET EUROPEEN

(11) **EP 1 365 775 B1**
(45) Date de publication et mention de la délivrance du brevet: **04.08.2010**
(21) Numéro de dépôt: 02716882.2
(22) Date de dépôt: 08.03.2002
(51) Int. Cl.: A61K 31/7048, A61K 9/50

(54) **SUSPENSION DE TELITHROMYCINE A GOUT MASQUE**
GESCHMACKSMASKIERTE TELITHROMYCINHALTIGE SUSPENSION
TELITHROMYCIN SUSPENSION WITH A MASKED TASTE

(30) Priorité: 09.03.2001 FR 0103234
(43) Date de publication de la demande: 03.12.2003
(73) Titulaire: ETHYPHARM, 78550 Houdan (FR)
(72) Inventeur: MEISSONNIER, Julien, F-67610 La Wantzenau (FR); LEBON, Christophe, F-28260 Rouvres (FR); SALLE, Sandrine, F-78980 Saint Illiers La Ville (FR)
(74) Mandataire: Ahner, Francis
(86) Numéro de dépôt international: PCT/FR2002/000835
(87) Numéro de publication internationale: WO 2002/072111

(56) Documents cités:
- EP-A- 0 680 967
- WO-A-01/14393
- US-A- 4 935 247

## Description

La présente invention concerne une suspension sèche reconstituable de télithromycine à goût masqué préparée à partir de granules et de granulés enrobés et apte à être utilisée par voie orale.

La télithromycine, issue d'une famille d'antibiotiques proche des macrolides, les kétolides, bloque également la synthèse protéique et possède un large spectre d'action notamment contre des germes résistants pathogènes du tractus respiratoire comme *Haemophilus influenzae, Moraxella catarrhalis,* voire également *Chlamydia pneumoniae, Legionella pneumophila* et *Mycoplasma pneumoniae,* et ceci en administration *per os.* Elle est par conséquent utilisée comme antibiotique dans le traitement des infections à germes sensibles (EP 680967).

Toutefois, la télithromycine comme de nombreux autres principes actifs, en particulier les macrolides, présente un goût désagréable, de sorte qu'il est indispensable de masquer son goût. On définit par masquage de goût, tout procédé permettant de retarder ou d'empêcher l'apparition d'un goût désagréable spécifique à la télithromycine lors de son administration par voie orale.

Dans le cas de formulations administrées sous forme liquide, de compositions conditionnées sous formes de flacons multidoses, et en particulier dans le cas de suspensions sèches destinées à une reconstitution extemporanée, appelées également suspensions sèches reconstituables, l'absence d'amertume doit être maintenue pendant un temps équivalent soit à la durée du traitement, soit à la durée d'utilisation du flacon. Le granule ou granulé actif enrobé utilisé dans de telles formulations devra donc être stable au contact d'une phase liquide aqueuse pendant un temps au moins égal à 24 heures. En pratique, ceci revient à empêcher la solubilisation du principe actif dans la phase liquide.

En général, le masquage du goût est réalisé par encapsulation du principe actif à l'intérieur d'une capsule ou par des techniques de microencapsulation dans lesquelles un enrobage polymérique est appliqué sur le principe actif (WO 92/11871).

L'une des solutions proposées consiste à enrober les particules de principe actif par un polymère cellulosique. Parmi ces polymères, on peut citer notamment l'éthylcellulose et l'hydroxypropylméthyl-cellulose.

Une autre solution consiste à enrober la particule de principe actif avec un polymère de type acrylique. Parmi ces polymères, on distingue les polymères pH dépendants, c'est-à-dire les polymères dont la solubilité dépend du pH et les polymères pH indépendants, c'est-à-dire les polymères insolubles dont les propriétés intrinsèques ne sont pas influencées par le pH du milieu.

Toutefois, même si le goût du principe actif présent dans les granules est masqué de façon satisfaisante, ces polymères interfèrent avec la libération du principe actif et nécessitent l'utilisation d'agents favorisant ou retardant la solubilisation du principe actif (GB 1 511 852; WO 91/16043).

De plus, les techniques et formules conventionnelles, bien que procurant un bon masquage de goût, ne permettent pas d'obtenir des membranes stables en suspension plus d'une journée.

Des microsphères matricielles ont également été stabilisées, mais elles nécessitent un enrobage supplémentaire pour atteindre la stabilité souhaitée; une stabilité correcte peut être obtenue en pH acide avec des acétates de cellulose, mais on observe un retard à la libération (EP 0293885). WO 01/14393 décrit des agglomérats sphériques de télithromycine entourés d'une couche de polymère. Ces agglomérats sont destinés à la préparation extemporanée de suspensions orales.

Aussi, existe-t-il encore un grand besoin d'avoir une formulation qui permette une libération immédiate de la télithromycine dans un milieu physiologique, sans qu'il y ait libération dans le milieu de la formulation, et qui présente une stabilité suffisante, c'est à dire une capacité à conserver le masquage du goût pendant une période au moins égale à 24 heures.

Or, les inventeurs ont trouvé de manière surprenante qu'une suspension sèche reconstituable comprenant des granules ou des granulés comportant un coeur contenant la télithromycine, ledit coeur étant enrobé, et des excipients appropriés pour avoir une suspension sèche reconstituable en milieu liquide permet d'isoler la télithromycine pendant une durée suffisante pour assurer la stabilité du masquage du goût lorsque la suspension sèche reconstituable incorporant ledit granule ou granulé enrobé est reconstituée par ajout d'un volume d'eau défini au moment de la première prise. Après absorption, on a une libération immédiate de la télithromycine.

En conséquence, la présente invention a pour objet une suspension sèche reconstituable de télithromycine caractérisée en ce qu'elle contient des granules ou des granulés comprenant:
- un coeur contenant la télithromycine éventuellement associée à au moins un composé cireux et éventuellement à au moins un polymère et/ou à au moins un agent liant, et
- au moins trois couches d'enrobage successives en partant du coeur,
lesdits granules ou granulés étant associés à des excipients parmi lesquels au moins un agent épaississant, au moins un agent conservateur et au moins un agent modulateur de pH.

Cette association de granules ou de granulés enrobés avec ce type d'excipients confère à la formulation reconstituée des caractéristiques organoleptiques particulières, et une stabilité microbiologique.

Dans un mode de réalisation avantageux de l'invention, les excipients sont sous forme de grains associés aux granules ou granulés.

Les couches successives d'enrobage en partant du coeur sont les suivantes :
- un enrobage fonctionnel polymérique (1) contenant éventuellement un composé cireux permettant une libération immédiate,
- un enrobage hydrophobe (2) contenant au moins un composé cireux, et
- un enrobage fonctionnel polymérique (3) contenant éventuellement un composé cireux qui peut avoir une structure différente de l'enrobage (1), mais qui a également une fonction de libération immédiate et conditionne le milieu de suspension.

Au sens de la présente invention, on entend par libération immédiate, une libération dont la cinétique n'est pas substantiellement modifiée par la formulation et/ou par les paramètres du procédé de fabrication, ce qui signifie que le profil de dissolution de la télithromycine dépend essentiellement de ses propriétés intrinsèques.

Au sens de la présente invention, conditionner le milieu de suspension signifie que les caractéristiques de la suspension reconstituée, obtenue à partir des grains excipients, sont choisies en fonction du profil de libération du granule ou granulé actif enrobé, *in vitro* ou après administration de ladite suspension reconstituée.

Dans un mode particulier de réalisation de l'invention, des couches supplémentaires peuvent être appliquées dont la composition est identique à celle des couches (1) et (3).

Un surenrobage destiné à masquer une éventuelle amertume liée aux composants de la troisième couche d'enrobage (3), qui ne modifie pas substantiellement les propriétés de libération des granules et des granulés, peut être appliqué.

Dans un mode particulièrement avantageux de l'invention, le coeur est un substrat neutre préférentiellement sphérique de granulométrie déterminée, à base d'amidon, de saccharose, d'éthylcellulose, de lactose ou de cire, sur lequel on applique en couche la télithromycine par pulvérisation d'une suspension ou d'une solution de ladite télithromycine, dans un solvant aqueux, organique ou dans un mélange en présence d'au moins un agent liant ou d'au moins un polymère ou d'au moins un composé cireux ou d'un mélange d'au moins deux de ces agents et éventuellement de lubrifiants.

Dans un autre mode de réalisation avantageux de l'invention, le coeur est la télithromycine elle-même, sous forme de cristal sphérique ou non, si sa granulométrie permet de réaliser directement un enrobage efficace. Sinon une application en couche (montage) de la télithromycine sera réalisé par pulvérisation d'une solution ou d'une suspension de ladite télithromycine en présence d'au moins un agent liant ou d'au moins un polymère ou d'au moins un composé cireux ou d'un mélange d'au moins deux de ces agents et éventuellement de lubrifiants et de solvants organiques ou d'eau.

Dans un autre mode de réalisation particulièrement avantageux de l'invention, le coeur est un granulé à base de télithromycine obtenu par granulation. Le granulé peut être obtenu par granulation humide ou en lit d'air fluidisé ou par cristallisation sphérique ou par émulsion-diffusion de solvant, préférentiellement en utilisant (a) des solutions de granulations à base de solutions organiques de composé(s) cireux en présence d'agents lubrifiants et de plastifiants ou (b) un polymère tel que l'hydroxypropylméthylcellulose. En outre, un montage de la télithromycine pourra être réalisé en utilisant ledit granulé comme support, par pulvérisation d'une solution ou suspension de la télithromycine dans des solvants organiques ou dans l'eau, en présence d'au moins un agent liant ou d'au moins un polymère ou d'au moins un composé cireux ou d'un mélange d'au moins deux de ces agents et éventuellement de lubrifiants.

Outre la télithromycine, le coeur peut contenir divers agents ; parmi ces agents, on trouve des agents insolubles, notamment le talc, le dioxyde de silicone, le dioxyde de titane, la silice, l'alumine, l'amidon et leurs mélanges; on trouve également des agents solubles, notamment le mannitol, le saccharose, le lactose, le dextrose, le chlorure de sodium, le sorbitol et leurs mélanges, le polyéthylène glycol ou des composés ampiphiles (stéarate de magnésium, polysorbates).

Le coeur peut contenir jusqu'à 100 % de télithromycine, de préférence entre 60 et 85 % en fonction du dosage de la formulation finale.

Le coeur contenant la télithromycine peut avoir n'importe quelle taille adaptée, mais de préférence la distribution de taille du coeur contenant la télithromycine présente une moyenne comprise entre 100 à 500 µm, la moyenne étant préférentiellement comprise entre 100 à 250 µm lorsque le coeur est un granulé ou le principe actif lui-même, et préférentiellement comprise entre 400 et 500 µm lorsque le coeur est un support neutre sur lequel le principe actif est appliqué en couche.

Selon l'invention, les enrobages (1) et (3) sont des enrobages fonctionnels, qui ont pour but de conférer une propriété de libération immédiate de la télithromycine ; ils sont constitués par des polymères classiquement connus de l'homme du métier pour conférer lesdites propriétés. On peut notamment citer les polyméthacrylates notamment ceux commercialisés sous le nom Eudragit^{®}E et plus particulièrement Eudragit^{®}E 100.

Les agents cireux utilisés peuvent être notamment choisis dans le groupe constitué par les cires, les cires Novata^{®}, les gélucires et suppocires, les macrogol glycériques, les acides gras (acide stéarique), les esters d'acides gras, le monostéarate de glycérol Précirol^{®}, Compritol^{®}.

Parmi ces composés cireux, on utilisera avantageusement les composés cireux hydrophobes et encore plus avantageusement des composés cireux hydrophobes présentant un faible HLB (balance hydrophile-lipophile) et possédant un point de fusion compris entre 35 et 53°C, préférentiellement entre 37 et 43°C. On peut citer, à titre non limitatif, les composés cireux commercialisés sous les noms de Gélucire^{®} 43/01 et de Novata^{®}AB.

Ces composés cireux peuvent être associés à du monostéarate de glycérol (GMS).

Ainsi, dans un mode de réalisation particulièrement avantageux de l'invention, on peut utiliser comme enrobages fonctionnels (1) et (3), un enrobage constitué avantageusement d'un mélange d'Eudragit^{®} E100 et éventuellement de composés cireux hydrophobes présentant un faible HLB (balance hydrophile-lipophile) et possédant un point de fusion compris entre 35 et 53°C, préférentiellement entre 37 et 43°C en présence de lubrifiants. On peut citer, à titre non limitatif, Gélucire^{®} 43/01 et Novata^{®}AB, éventuellement associés à du monostéarate de glycérol (GMS).

Le taux d'enrobage pour l'enrobage (1) (calculé en pourcentage (p/p) de matière sèche appliquée sur le substrat de départ) est avantageusement compris entre 5 et 100% et préférentiellement entre 30 et 60%.

L'enrobage hydrophobe (2) a pour but d'augmenter la stabilité du grain en suspension. Il est constitué à base d'une solution de composés cireux dans un solvant et comprend éventuellement un agent de lubrification comme par exemple du talc ou de la silice colloïdale hydrophobe. Le taux d'enrobage pour ce second enrobage (calculé en pourcentage (p/p) de matière sèche appliquée sur le substrat de départ) est avantageusement compris entre 5 et 100% et préférentiellement entre 10 et 80%.

Ainsi, cet enrobage hydrophobe (2) comprend de manière avantageuse un composé cireux ou une association de composés cireux hydrophobes à faible HLB et possédant un point de fusion compris entre 35 et 53°C, préférentiellement 37 et 43°C dans un solvant. On peut citer notamment Gélucire^{®} 43/01, Gélucire^{®} 53/01, Novata^{®}AB, le monostéarate de glycérol et leurs mélanges. L'enrobage fonctionnel polymérique (3) qui a des fonctions de libération complémentaires à celles de l'enrobage (1) est soit identique, soit analogue audit enrobage (1), mais présente les mêmes propriétés vis à vis de la libération de la télithromycine et conditionne le milieu de suspension. Le taux d'enrobage au niveau de ce troisième enrobage (calculé en pourcentage (p/p) de matière sèche appliquée sur le substrat de départ) est avantageusement compris entre 5 et 200% et préférentiellement entre 40 et 100%.

Dans l'hypothèse où l'enrobage (3) possède un goût prononcé dû aux excipients qu'il comporte, alors un surenrobage à base d'Eudragits^{®} RL30D et RS30D ou de leurs mélanges, en présence de plastifiants et de lubrifiants, est appliqué. Le taux d'enrobage à ce niveau sera avantageusement compris entre 0 et 15% et préférentiellement entre 0 et 5%.

Les agents de lubrification (agents lubrifiants) sont avantageusement choisis dans le groupe comprenant le talc, la silice hydrophobe colloïdale et le monostéarate de glycérol.

Les plastifiants sont avantageusement choisis dans le groupe constitué par le dibutylsébaccate, le triéthylcitrate, le diéthylphtalate, l'acétyltriéthylcitrate, l'acétyltributylcitrate, le monostéarate de glycérol (GMS) et le Myvacet®.

Les granules et les granulés enrobés utilisés pour préparer la suspension sèche reconstitubale de l'invention sont préparés selon un procédé qui comprend la réalisation du coeur ou support et inclut éventuellement une étape complémentaire de montage.

Le procédé peut avantageusement comprendre les étapes suivantes:
- application de la télithromycine solubilisée sur le support, en présence de composés cireux préférentiellement hydrophobes et/ou de polymères, et d'au moins un agent de lubrification dans un solvant, ou un mélange de solvants,
- application d'un premier enrobage, enrobage fonctionnel polymérique (1) et éventuellement de composés cireux, ledit enrobage permettant une libération immédiate,
- application d'un second enrobage, enrobage hydrophobe (2) contenant au moins un composé cireux ou une association de composés cireux,
- application d'un troisième enrobage, enrobage fonctionnel polymérique (3) et éventuellement de composés cireux, ledit enrobage pouvant avoir une structure différente de celle de l'enrobage (1), mais présentant également une fonction de libération immédiate, et éventuellement
- séchage des granules ou des granulés ainsi obtenus.

Les solvants d'enrobage sont ceux classiquement utilisés par l'homme du métier. On peut citer à titre d'exemples l'eau, le chlorure de méthylène, l'éthanol, l'isopropanol et leurs mélanges.

Ce procédé est réalisé en lit d'air fluidisé ou par tout autre procédé industriel similaire connu de l'homme du métier.

L'opération de séchage peut être réalisée en lit d'air fluidisé, en sécheur rotatif sous vide ou par toute technique équivalente permettant d'enlever les solvants résiduels.

Lorsque c'est nécessaire, le procédé comprend en outre l'application de couches supplémentaires identiques aux couches (1) et (3).

Dans un mode de réalisation avantageux de réalisation de l'invention les grains d'excipients contiennent, outre un agent épaississant, un agent conservateur et un agent modulateur de pH, d'autres excipients choisis parmi ceux classiquement utilisés par l'homme de l'art pour réaliser ces formulations. On peut citer parmi ces excipients les édulcorants, les agents de charge, les compositions aromatiques, les colorants, les agents tensioactifs et les antioxydants.

Cette suspension peut être obtenue de plusieurs manières:
- par adjonction simple au grain actif contenant la télithromycine des excipients sous forme de mélange de poudre,
- par adjonction au grain actif contenant la télithromycine d'un granulé sec d'excipients. Dans ce cas, les excipients sont des granulés préférentiellement obtenus par granulation humide,
- par adjonction au grain actif contenant la télithromycine des excipients montés sur le grain actif par un procédé d'enrobage réalisé avantageusement en lit d'air fluidisé.

Au titre d'épaississant, on peut citer tous les épaississants connus de l'homme du métier, notamment ceux choisis dans le groupe comprenant les gommes comme le xanthane, le guar et la gomme adragante, le silicate de magnésium et leurs associations, l'alginate de sodium, l'alginate de propylène glycol, les composés cellulosiques tels l'hydroxyéthyle cellulose, l'hydroxypropyle cellulose, la méthyle cellulose, la carboxyméthyle cellulose, les carbopols, la gélatine, les poloxamères, ou les associations de ces composés et les carraghénanes.

Au titre d'agent ajusteur de pH, on peut citer avantageusement ceux choisis dans le groupe comprenant l'acide citrique, la soude, le citrate de sodium, le citrate trisodique ou tout composé pharmaceutiquement acceptable ayant la capacité de tamponner une solution aqueuse.

Au titre d'agent conservateur, on peut citer ceux choisis dans le groupe comprenant le sorbate de potassium ou de sodium, le benzoate de sodium, l'azorubine, le bronopol, l'acide éthylène diamine tétraacétique (EDTA), les p-hydroxybenzoate (parabènes) de méthyle, d'éthyle, de propyle et de butyle ainsi que leurs sels, utilisés seuls ou en mélange, l'acide propionique, les sulfites et le crésol.

La suspension peut en outre contenir un ou plusieurs édulcorant(s) comme les sels de saccharine et/ou l'acésulfame de potassium, ou tout autre édulcorant connu de l'homme du métier tel que l'aspartam, le saccharose et ses dérivés, le tréhalose, le glycyrrhizinate de sodium ou leurs mélanges, un agent opacifiant comme Opadry^{®}OYB ou les oxydes de titane, et des agents de capture des produits tels que les cyclodextrines dont les quantités seront adaptées en fonction de la taille de la molécule et de la fonction à isoler.

La suspension peut également contenir une ou plusieurs composition(s) aromatique(s) et un agent de charge, en particulier des polyols, par exemple le sorbitol (Neosorb®), le xylitol et le lactitol.

Le grain d'excipient peut être obtenu par un procédé de granulation humide ou tout autre procédé industriel similaire connu de l'homme du métier. Il peut être notamment obtenu par réalisation d'une solution hydro-alcanolique des édulcorants et/ou des conservateurs qui servira de liquide de mouillage à un mélange d'agent de charge tel le sorbitol, d'agent épaississant, d'agent opacifiant, d'agent ajusteur de pH, éventuellement des compositions aromatiques, l'agent de charge ayant pour fonction de créer une masse suffisante pour la granulation. Tout autre excipient remplissant la même fonction pourra également être utilisé.

Une autre alternative consiste à monter les excipients sur le grain actif par toute technique connue de l'homme du métier, notamment en lit d'air fluidisé.

Au moment de la première prise du médicament, la suspension est préparée par ajout d'une quantité d'eau définie (par exemple en volume, ou par un trait de jauge sur le flacon), directement dans le flacon contenant le mélange sec final.

Les grains excipients ainsi préparés permettent une reconstitution rapide de la suspension , qui ne nécessite qu'une agitation manuelle par retournement pour homogénéiser la préparation ; en outre la suspension obtenue présente une bonne stabilité bactériologique et une stabilité du masquage supérieure à 7 jours et indépendante du pH de la suspension. Elle est particulièrement utile en pédiatrie et en gériatrie.

Le pH de la suspension est ajusté de manière à être compris entre 5,5 et 10, de préférence entre 8,5 et 10.

Grâce à la présence des agents cireux, la stabilité du masquage des suspensions est améliorée. Les agents cireux permettent en outre de diminuer la quantité de polymères utilisés pour l'enrobage, donc la toxicité induite par lesdits polymères.

L'invention et les avantages qui en découlent, ressortiront mieux des exemples de réalisation qui suit.

### Exemple 1: préparation du grain actif sans eau

### 1.1. Préparation du grain actif:

La télithromycine utilisée possédant une granulométrie suffisamment élevée, le montage est réalisé sur la télithromycine seule.

### • Etape 1: montage

Une solution à base de télithromycine -Novata^{®} AB - Talc M 10 (57,1%-28,6%-14,3%) dans un mélange de chlorure de méthylène - éthanol (50%-50% en poids) est pulvérisée sur le grain de télithromycine.

La concentration en sec dans le chlorure de méthylène - éthanol est égale à 43,75% en poids et le rapport sec pulvérisé/substrat est égal à 131,3% en poids.

### • Etape 2: enrobage (1) = enrobage fonctionnel polymérique

Une solution à base d'Eudragit^{®} E 100 - Novata^{®} AB - Talc M 10 (51,4%-5,7%-42,9%) dans le chlorure de méthylène est pulvérisée sur le granulé obtenu à l'étape 1.

La concentration en sec dans le chlorure de méthylène est égale à 19,4% en poids et le rapport sec pulvérisé/substrat est égal à 52,5% en poids.

### • Etape 3: enrobage (2) = enrobage hydrophobe

Une solution à base de Novata^{®} AB et de monostéarate de glycérol (GMS) - Talc M 10 (51,4%-5,7%-42,9%) dans un mélange de chlorure de méthylène - éthanol (70-30) est pulvérisée sur le granulé obtenu à l'étape 2.

La concentration en sec dans le chlorure de méthylène - éthanol est égale à 19,4% en poids et le rapport sec pulvérisé/substrat est égal à 35% en poids.

### • Etape 4: enrobage (3) = enrobage fonctionnel polymérique

Une solution à base d'Eudragit^{®} E 100 - Novata^{®} AB - Talc M 10 (45,7%-11,4%-42,9%) dans le chlorure de méthylène est pulvérisée sur le granulé obtenu à l'étape 3.

La concentration en sec dans le chlorure de méthylène est égale à 19,4% en poids et le rapport sec pulvérisé/substrat est égal à 87,5% en poids.

### 1.2. Préparation du grain pour suspension:

Les excipients sont mélangés dans des quantités permettant d'avoir les proportions suivantes:

| Excipient | Quantité (g) |
|---|---|
| Carraghénane | 0,933 |
| Opacifiant | 0,933 |
| Sorbitol | 7,6 |
| Composition aromatique | 0,477 |
| Acide citrique | 0,013 |
| Nipasept de sodium | 0,300 |
| Saccharinate de sodium | 0,081 |
| Acésulfame de potassium | 0,020 |

1.3. Répartition et reconstitution de la suspension: 30,5% de grains d'excipients et 69,5% de grains de télithromycine sont introduits dans le conditionnement final (par mélange puis simple alimentation ou double alimentation sans mélange préalable). Le flacon sera rempli en fonction de la dose de TLM à administrer et complété au niveau, au moment de l'utilisation, par de l'eau minérale. La suspension reconstituée est stable pendant au moins 7 jours.

### Exemple 2: préparation de grain actif en présence d'eau

Ce procédé permet de diminuer la granulométrie des particules de 200 µm.
- Etape 1: elle est identique à l'étape 1 décrite dans l'exemple 1 ci-dessus.
- Etape 2: enrobage (1) = enrobage fonctionnel polymérique

Une solution à base d'Eudragit^{®} E 100 - Novata^{®} AB - Talc M 10 (52,9%-5,9%-41,2%) dans un mélange chlorure de méthylène/eau (9/1) est pulvérisée sur le granulé obtenu à l'étape 1.

La concentration en sec dans le chlorure de méthylène/eau est égale à 18,9% en poids et le rapport sec pulvérisé/substrat est égal à 43,7% en poids.

### • Etape 3: enrobage (2) = enrobage hydrophobe

Une solution à base de Novata^{®} AB et de monostéarate de glycérol (GMS) - Talc M 10 (52,9%-5,9%-41,2%) dans un mélange de chlorure de méthylène-éthanol-eau (66,5-28,5-5) est pulvérisée sur le granulé obtenu à l'étape 2.

La concentration en sec dans le chlorure de méthylène-éthanol-eau est égale à 17,9% en poids et le rapport sec pulvérisé/substrat est égal à 35% en poids.

### • Etape 4: enrobage (3) = enrobage fonctionnel polymérique

Une solution à base d'Eudragit^{®} E 100 - Novata^{®} AB - Talc M 10 (53,3%-13,3%-33,3%) dans un mélange chlorure de méthylène-eau (9/1) est pulvérisée sur le granulé obtenu à l'étape 3.

La concentration en sec dans le chlorure de méthylène est égale à 16,7% en poids et le rapport sec pulvérisé/substrat est égal à 75% en poids.

### Exemple 3: tests de solubilité et de stabilité

La stabilité de la suspension est évaluée en terme de pH, de masquage de goût et de dosage de la télithromycine relarguée.

La stabilité des granulés est évaluée en terme de produits de dégradation, cinétique de dissolution, goût et solvants résiduels.

La suspension selon l'invention est stable pendant au moins 2 mois et présente un masquage du goût durable.

Le profil de dissolution mesuré à pH 1 à 100 rpm est représenté dans le tableau ci-dessous :

| Temps (minutes) | Profil de dissolution de la suspension (% de télithromycine dissoute) | |
|---|---|---|
| | Après reconstitution | Au bout de 5 jours |
| 5 | 70,0 | 62,8 |
| 10 | 88,8 | 80,2 |
| 30 | 93,9 | 88,2 |
| 60 | 94,0 | 90,3 |

## Revendications

1. Suspension sèche reconstituable de télithromycine **caractérisée en ce qu'**elle contient des granules ou des granulés comprenant:
- un coeur contenant la télithromycine éventuellement associée à au moins un composé cireux et éventuellement à au moins un polymère et/ou à un agent liant, et
- au moins trois couches d'enrobage successives en partant du coeur, **caractérisée en ce que** les couches successives d'enrobage en partant du coeur sont les suivantes:
• un enrobage fonctionnel polymérique (1) contenant éventuellement un composé cireux, permettant une libération immédiate,
• un enrobage hydrophobe (2) contenant au moins un composé cireux, et
• un enrobage fonctionnel polymérique (3) contenant éventuellement un composé cireux, qui peut avoir une structure différente de l'enrobage (1), mais qui a également une fonction de libération immédiate et conditionne le milieu de suspension;
lesdits granules ou granulés étant associés à des excipients parmi lesquels au moins un agent épaississant, au moins un agent conservateur et au moins un agent modulateur de pH.

2. Suspension sèche reconstituable selon la revendication 1, **caractérisée en ce que** les excipients sont associés sous forme de grains.

3. Suspension sèche reconstituable selon l'une quelconque des revendications 1 et 2 **caractérisée en ce que** le coeur est un substrat neutre sur lequel la télithromycine est appliquée en couche.

4. Suspension sèche reconstituable selon l'une quelconque des revendications 1 et 2 **caractérisée en ce que** le coeur est la télithromycine elle-même, sous forme de cristal sphérique ou non.

5. Suspension sèche reconstituable selon l'une quelconque des revendications 1 et 2 **caractérisée en ce que** le coeur est un granulé à base de télithromycine obtenu par granulation, par cristallisation sphérique ou par émulsion-diffusion de solvant.

6. Suspension sèche reconstituable selon l'une quelconque des revendications 1 à 5 **caractérisée en ce que** outre la télithromycine, le coeur contient divers agents.

7. Suspension sèche reconstituable selon l'une quelconque des revendications 1 à 6 **caractérisée en ce que** le coeur contient jusqu'à 100 % de télithromycine, de préférence entre 60 et 85 % en fonction du dosage de la formulation finale.

8. Suspension sèche reconstituable selon la revendication 1 **caractérisée en ce que** la distribution de taille du coeur contenant la télithromycine présente une moyenne comprise entre 100 à 500 µm.

9. Suspension sèche reconstituable selon l'une quelconque des revendications 4 et 5 **caractérisée en ce que** la distribution de taille du coeur contenant la télithromycine présente une moyenne comprise entre 100 à 250 µm.

10. Suspension sèche reconstituable selon la revendication 3 **caractérisée en ce que** la distribution de taille du coeur contenant la télithromycine présente une moyenne comprise entre 400 et 500 µm.

11. Suspension sèche reconstituable selon l'une quelconque des revendications 1 à 10 **caractérisée en ce que** les enrobages fonctionnels (1) et (3) conférant des propriétés de libération immédiate, sont des enrobages à base de polyméthacrylate.

12. Suspension sèche reconstituable selon la revendication 11 **caractérisée en ce que** le polyméthacrylate est de l'Eudragit^{®}E 100.

13. Suspension sèche reconstituable selon l'une quelconque des revendications 1 à 12 **caractérisée en ce que** les composés cireux sont choisis dans le groupe comprenant les cires, les gélucires et suppocires, les macrogol glycériques, les acides gras et les esters d'acides gras.

14. Suspension sèche reconstituable selon la revendication 13 **caractérisée en ce que** les composés cireux sont des composés cireux hydrophobes présentant un faible HLB (balance hydrophile-lipophile) et possédant un point de fusion compris entre 35 et 53°C, préférentiellement entre 37 et 43°C.

15. Suspension sèche reconstituable selon l'une quelconque des revendications 13 et 14 **caractérisée en ce que** les composés cireux sont associés à du monostéarate de glycérol.

16. Suspension sèche reconstituable selon l'une quelconque des revendications 1 à 15 **caractérisée en ce que** les enrobages fonctionnels (1) et (3) sont constitués d'un mélange d'Eudragit^{®} E100 et de composés cireux hydrophobes présentant un faible HLB (balance hydrophile-lipophile) et possédant un point de fusion compris entre 35 et 53°C, préférentiellement entre 37 et 43°C en présence de lubrifiants.

17. Suspension sèche reconstituable selon l'une quelconque des revendications 1 à 16 **caractérisée en ce que** le taux d'enrobage pour l'enrobage (1) est compris entre 5 et 100%, préférentiellement entre 30 et 60%, le taux d'enrobage pour l'enrobage (2) est compris entre 5 et 100%, et préférentiellement entre 20 et 80% et le taux d'enrobage pour l'enrobage (3) est compris entre 5 et 200%, préférentiellement entre 40 et 100%.

18. Suspension sèche reconstituable selon l'une quelconque des revendications 1 à 17 **caractérisée en ce qu'**elle contient des agents de lubrification choisis dans le groupe comprenant le talc, la silice hydrophobe colloïdale et le monostéarate de glycérol.

19. Suspension sèche reconstituable selon l'une quelconque des revendications 1 à 18 **caractérisée en ce que** les plastifiants sont choisis dans le groupe constitué par le dibutylsébaccate, le triéthylcitrate, le diéthylphtalate, l'acétyltriéthylcitrate, l'acétyl-tributylcitrate et le monostéarate de glycérol.

20. Suspension sèche reconstituable selon l'une quelconque des revendications 1 à 19 **caractérisée en ce que** les grains d'excipients contiennent, outre un agent épaississant, un agent conservateur et un agent modulateur de pH, des excipients choisis dans le groupe comprenant les édulcorants, les agents de charge, les compositions aromatiques, les colorants, les agents tensioactifs et les antioxydants.

21. Suspension sèche reconstituable selon l'une quelconque des revendications 1 à 20 **caractérisée en ce que** l'épaississant est choisi dans le groupe comprenant les gommes comme le xanthane, le guar et la gomme adragante, le silicate de magnésium et leurs associations, l'alginate de sodium, l'alginate de propylène glycol, les composés cellulosiques tels l'hydroxyéthyle cellulose, l'hydroxypropyle cellulose, la méthyle cellulose, la carboxyméthyle cellulose, les carbopols, la gélatine, les poloxamères, ou les associations de ces composés et les carraghénanes.

22. Suspension sèche reconstituable selon l'une quelconque des revendications 1 à 21 **caractérisée en ce que** l'agent ajusteur de pH est choisi dans le groupe comprenant l'acide citrique, la soude, le citrate de sodium, le citrate trisodique.

23. Suspension sèche reconstituable selon l'une quelconque des revendications 1 à 22 **caractérisée en ce que** l'agent conservateur, est choisi dans le groupe comprenant le sorbate de potassium ou de sodium, le benzoate de sodium, l'azorubine, le bronopol, l'acide éthylène diamine tétraacétique (EDTA), les p-hydroxybenzoate (parabènes) de méthyle, d'éthyle, de propyle et de butyle ainsi que leurs sels, utilisés seuls ou en mélange, l'acide propionique, les sulfates et le crésol.

24. Suspension sèche reconstituable selon l'une quelconque des revendications 1 à 23 **caractérisée en ce que** le pH de la suspension est compris entre 5,5 et 10, de préférence entre 8,5 et 10.

## Claims

1. A dry reconstitutable telithromycin suspension, **characterised in that** it contains granules or granulates comprising
- a core containing telithromycin optionally associated with at least one wax-like compound and optionally with at least one polymer and/or with one binding agent, and
- at least three successive layers of coating starting out from the core, **characterised in that** the successive layers of coating starting out from the core are the following:
- - a functional polymer coating (1) optionally containing a wax-like compound, enabling immediate release.
- - a hydrophobic coating (2) containing at least one wax-like compound, and
- - a functional polymer coating (3) optionally containing a wax-like compound, which can have a different structure from the coating (1), but which also has an immediate release function and conditions the suspension medium;
said granules or granulates being associated with excipients including at least one thickening agent, at least one preservative agent and at least one pH modulator agent.

2. The dry reconstitutable suspension as claimed in Claim 1, **characterised in that** the excipients are associated in the form of grains.

3. The dry reconstitutable suspension as claimed in any of Claims 1 and 2, **characterised in that** the core is a neutral substrate on which telithromycin is applied in layers.

4. The dry reconstitutable suspension as claimed in any of Claims 1 and 2, **characterised in that** the core is telithromycin itself, in the form of crystal, spherical or not.

5. The dry reconstitutable suspension as claimed in any of Claims 1 and 2, **characterised in that** the core is a granule based on telithromycin obtained by granulation, by spherical crystallisation or by emulsion-diffusion of solvent.

6. The dry reconstitutable suspension as claimed in any of Claims 1 to 5, **characterised in that** apart from telithromycin, the core contains various agents.

7. The dry reconstitutable suspension as claimed in any one of Claims 1 to 6, **characterised in that** the core contains up to 100% telithromycin, preferably between 60 and 85% as a function of the dosage of the final formulation.

8. The dry reconstitutable suspension as claimed in Claim 1, **characterised in that** the size distribution of the core containing telithromycin has an average of between 100 to 500 µm.

9. The dry reconstitutable suspension as claimed in any of Claims 4 and 5, **characterised in that** the size distribution of the core containing telithromycin has an average of between 100 to 250 µm.

10. The dry reconstitutable suspension as claimed in Claim 3, **characterised in that** the size distribution of the core containing telithromycin has an average of between 400 and 500 µm.

11. The dry reconstitutable suspension as claimed in any of Claims 1 to 10, **characterised in that** the functional coatings (1) and (3) imparting immediate release properties are coatings based on polymethacrylate.

12. The dry reconstitutable suspension as claimed in Claim 11, **characterised in that** the polymethacrylate is Eudragit^{®} E 100.

13. The dry reconstitutable suspension as claimed in any of Claims 1 to 12, **characterised in that** the wax-like compounds are selected from the group constituted by waxes, gelucires and suppocires, glyceric macrogol, fatty acids and fatty acid esters.

14. The dry reconstitutable suspension as claimed in Claim 13, **characterised in that** the wax-like compounds are wax-like hydrophobic compounds exhibiting low HLB (hydrophilic-lipophilic balance) and having a melting point between 35 and 53°C, preferably between 37 and 43°C.

15. The dry reconstitutable suspension as claimed in any of Claims 13 to 14, **characterised in that** the wax-like compounds are associated with glycerol monostearate.

16. The dry reconstitutable suspension as claimed in any of Claims 1 to 15, **characterised in that** the functional coatings (1) and (3) are constituted by a mixture of Eudragit^{®} E100 and wax-like hydrophobic compounds exhibiting low HLB (hydrophilic-lipophilic balance) and having a melting point between 35 and 53°C, preferably between 37 and 43°C in the presence of lubricants.

17. The dry reconstitutable suspension as claimed in any of Claims 1 to 16, **characterised in that** the rate of coating for the coating (1) is between 5 and 100%, preferably between 30 and 60%, the rate of coating for the coating (2) is between 5 and 100%, and preferably between 20 and 80% and the rate of coating for the coating (3) is between 5 and 200%, preferably between 40 and 100%.

18. The dry reconstitutable suspension as claimed in any of Claims 1 to 17, **characterised in that** the lubrication agents are selected from the group comprising talc, hydrophobic colloidal silica and glycerol monostearate.

19. The dry reconstitutable suspension as claimed in any of Claims 1 to 18, **characterised in that** the plasticisers are selected from the group constituted by dibutyl sebaccate, triethyl citrate, diethyl phthalate, acetyl triethyl citrate, acetyl tributyl citrate and glycerol monostearate.

20. The dry reconstitutable suspension as claimed in any of Claims 1 to 19, **characterised in that** the excipient grains contain, apart from a thickening agent, preservative agent and a pH modulator agent, excipients selected from the group comprising sweeteners, fillers, aromatic compositions, coloring agents, surfactants and antioxidants.

21. The dry reconstitutable suspension as claimed in any of Claims 1 to 20, **characterised in that** the thickener is selected from the group comprising gums such as xanthan, guar and gum tragacanth, magnesium silicate and the associations thereof, sodium alginate, propylene glycol alginate, cellulose compounds such as hydroxyethyl cellulose, hydxoxypropyl cellulose, methyl cellulose, carboxymethyl cellulose, carbopols, gelatine, poloxamers, or associations of these compounds and carrageenans.

22. The dry reconstitutable suspension as claimed in any of Claims 1 to 21, **characterised in that** the pH adjuster agent is selected from the group comprising citric acid, soda, sodium citrate, trisodium citrate.

23. The dry reconstitutable suspension as claimed in any of Claims 1 to 22, **characterised in that** the preservative agent is selected from the group comprising potassium or sodium sorbate, sodium benzoate, azorubin, bronopol, ethylene diamine tetraacetic acid (EDTA), methyl, ethyl, propyl and butyl p-hydroxybenzoate (parabens), as well as the salts thereof, used singly or in a mixture, propionic acid, sulphates and cresol.

24. The dry reconstitutable suspension as claimed in any of Claims 1 to 23, **characterised in that** the pH of the suspension is between 5.5 and 10, preferably between 8.5 and 10.

## Patentansprüche

1. Rekonstituierbare trockene Telithromycin-Suspension, **dadurch gekennzeichnet, dass** sie Körnchen oder Granulat enthält, umfassend:
- einen Kern, der das Telithromycin enthält, gegebenenfalls in Verbindung mit mindestens einer wachsartigen Verbindung und gegebenenfalls mit mindestens einem Polymer und/oder einem Bindemittel, und
- mindestens drei aufeinander folgende Überzugsschichten, die von dem Kern ausgehen, **dadurch gekennzeichnet, dass** die aufeinander folgenden Überzugsschichten, die von dem Kern ausgehen, die folgenden sind:
- - ein funktioneller Polymerüberzug (1), der gegebenenfalls eine wachsartige Verbindung enthält und der eine sofortige Freisetzung ermöglicht,
- - ein hydrophober Überzug (2), der mindestens eine wachsartige Verbindung enthält, und
- - ein funktioneller Polymerüberzug (3), der gegebenenfalls eine wachsartige Verbindung enthält und der eine von dem Überzug (1) unterschiedliche Struktur aufweisen kann, der jedoch ebenfalls eine sofortige Freisetzungsfunktion aufweist und in dem Suspensionsmedium aufbereitet ist;
wobei die Körnchen oder das Granulat mit Exzipienten verbunden sind, darunter mindestens ein Verdickungsmittel, mindestens ein Konservierungsmittel und mindestens ein Mittel zur Einstellung des pH-Werts.

2. Rekonstituierbare trockene Suspension nach Anspruch 1, **dadurch gekennzeichnet, dass** die Exzipienten in der Form von Körnern verbunden sind.

3. Rekonstituierbare trockene Suspension nach einem der Ansprüche 1 und 2, **dadurch gekennzeichnet, dass** der Kern ein neutrales Substrat ist, auf dem das Telithromycin als Schicht aufgebracht ist.

4. Rekonstituierbare trockene Suspension nach einem der Ansprüche 1 und 2, **dadurch gekennzeichnet, dass** der Kern das Telithromycin selbst ist, in der Form eines sphärischen oder nicht sphärischen Kristalls.

5. Rekonstituierbare trockene Suspension nach einem der Ansprüche 1 und 2, **dadurch gekennzeichnet, dass** der Kern ein Granulat auf Telithromycin-Basis ist, das durch Granulation, durch sphärische Kristallisation oder durch Emulsion/Diffusion von Lösungsmittel erhalten wird.

6. Rekonstituierbare trockene Suspension nach einem der Ansprüche 1 bis 5, **dadurch gekennzeichnet, dass** der Kern außer dem Telithromycin verschiedene Mittel enthält.

7. Rekonstituierbare trockene Suspension nach einem der Ansprüche 1 bis 6, **dadurch gekennzeichnet, dass** der Kern bis zu 100 % Telithromycin, vorzugsweise zwischen 60 und 85 %, je nach der Dosierung der Endformulierung, enthält.

8. Rekonstituierbare trockene Suspension nach Anspruch 1, **dadurch gekennzeichnet, dass** die Größenverteilung des Kerns, der das Telithromycin enthält, einen Mittelwert aufweist, der zwischen 100 und 500 µm liegt.

9. Rekonstituierbare trockene Suspension nach einem der Ansprüche 4 und 5, **dadurch gekennzeichnet, dass** die Größenverteilung des Kerns, der das Telithromycin enthält, einen Mittelwert aufweist, der zwischen 100 und 250 µm liegt.

10. Rekonstituierbare trockene Suspension nach Anspruch 3, **dadurch gekennzeichnet, dass** die Größenverteilung des Kerns, der das Telithromycin enthält, einen Mittelwert aufweist, der zwischen 400 und 500 µm liegt.

11. Rekonstituierbare trockene Suspension nach einem der Ansprüche 1 bis 10, **dadurch gekennzeichnet, dass** die funktionellen Überzüge (1) und (3), die Eigenschaften zur sofortigen Freisetzung verleihen, Überzüge auf Polymethacrylat-Basis sind.

12. Rekonstituierbare trockene Suspension nach Anspruch 11, **dadurch gekennzeichnet, dass** das Polymethacrylat Eudragit^{®} E 100 ist.

13. Rekonstituierbare trockene Suspension nach einem der Ansprüche 1 bis 12, **dadurch gekennzeichnet, dass** die wachsartigen Verbindungen aus der Gruppe ausgewählt sind, die Wachse, Gelucire und Suppocire, Macrogol-Glycerole, Fettsäuren und Fettsäureester umfasst.

14. Rekonstituierbare trockene Suspension nach Anspruch 13, **dadurch gekennzeichnet, dass** die wachsartigen Verbindungen hydrophobe wachsartige Verbindungen sind, die ein niedriges HLG (Hydrophile-Lipophile-Gleichgewicht) aufweisen und über einen Schmelzpunkt verfügen, der zwischen 35 und 53 °C, vorzugsweise zwischen 37 und 43 °C liegt.

15. Rekonstituierbare trockene Suspension nach einem der Ansprüche 13 und 14, **dadurch gekennzeichnet, dass** die wachsartigen Verbindungen mit Glycerolmonostearat verbunden sind.

16. Rekonstituierbare trockene Suspension nach einem der Ansprüche 1 bis 15, **dadurch gekennzeichnet, dass** die funktionellen Überzüge (1) und (3) aus einem Gemisch von Eudragit^{®} E 100 und hydrophoben wachsartigen Verbindungen, die ein niedriges HLG (Hydrophile-Lipophile-Gleichgewicht) aufweisen und über einen Schmelzpunkt verfügen, der zwischen 35 und 53 °C, vorzugsweise zwischen 37 und 43 °C liegt, in Anwesenheit von Gleitmitteln bestehen.

17. Rekonstituierbare trockene Suspension nach einem der Ansprüche 1 bis 16, **dadurch gekennzeichnet, dass** der Überzugsanteil für den Überzug (1) zwischen 5 und 100 %, vorzugsweise zwischen 30 und 60 % liegt, der Überzugsanteil für den Überzug (2) zwischen 5 und 100 % und vorzugsweise zwischen 20 und 80 % liegt und der Überzugsanteil für den Überzug (3) zwischen 5 und 200 %, vorzugsweise zwischen 40 und 100 % liegt.

18. Rekonstituierbare trockene Suspension nach einem der Ansprüche 1 bis 17, **dadurch gekennzeichnet, dass** sie Gleitmittel enthält, die aus der Gruppe ausgewählt sind, die Talkum, hydrophobes kolloidales Siliciumdioxid und Glycerolmonostearat umfasst.

19. Rekonstituierbare trockene Suspension nach einem der Ansprüche 1 bis 18, **dadurch gekennzeichnet, dass** die Weichmacher aus der Gruppe ausgewählt sind, die aus Dibutylsebacat, Triethylcitrat, Diethylphthalat, Acetyltriethylcitrat, Acetyltributylcitrat und Glycerolmonostearat besteht.

20. Rekonstituierbare trockene Suspension nach einem der Ansprüche 1 bis 19, **dadurch gekennzeichnet, dass** die Körner der Exzipienten außer einem Verdickungsmittel, einem Konservierungsmittel und einem Mittel zur Einstellung des pH-Werts Exzipienten enthalten, die aus der Gruppe ausgewählt sind, die Süßstoffe, Füllstoffe, Aromastoffe, Farbstoffe, Tenside und Antioxidationsmittel umfasst.

21. Rekonstituierbare trockene Suspension nach einem der Ansprüche 1 bis 20, **dadurch gekennzeichnet, dass** das Verdickungsmittel aus der Gruppe ausgewählt ist, die Gummen, wie Xanthan-, Guar- und Tragantgummi, Magnesiumsilikat und dessen Derivate, Natriumalginat, Propylenglykolalginat, Celluloseverbindungen, wie Hydroxyethylcellulose, Hydroxypropylcellulose, Methylcellulose, Carboxymethylcellulose, Carbopole, Gelatine, Poloxamere oder die Derivate dieser Verbindungen und Carrageene umfasst.

22. Rekonstituierbare trockene Suspension nach einem der Ansprüche 1 bis 21, **dadurch gekennzeichnet, dass** das Mittel zur Einstellung des pH-Werts aus der Gruppe ausgewählt ist, die Citronensäure, Soda, Natriumcitrat, Trinatriumcitrat umfasst.

23. Rekonstituierbare trockene Suspension nach einem der Ansprüche 1 bis 22, **dadurch gekennzeichnet, dass** das Konservierungsmittel aus der Gruppe ausgewählt ist, die Kalium- oder Natriumsorbat, Natriumbenzoat, Azorubin, Bronopol, Ethylendiamintetraessigsäure (EDTA), Methyl-p-hydroxybenzoat, Ethyl-p-hydroxybenzoat (Parabene), Propyl-p-hydroxybenzoat und Butyl-p-hydroxybenzoat sowie deren Salze, die für sich oder im Gemisch verwendet werden, Propionsäure, Sulfate und Kresol umfasst.

24. Rekonstituierbare trockene Suspension nach einem der Ansprüche 1 bis 23, **dadurch gekennzeichnet, dass** der pH-Wert der Suspension zwischen 5,5 und 10, vorzugsweise zwischen 8,5 und 10 liegt.
